# EUROPÄISCHE PATENTSCHRIFT

(11) **EP 0 693 120 B1**
(45) Veröffentlichungstag und Bekanntmachung des Hinweises auf die Patenterteilung: **10.02.1999**
(21) Anmeldenummer: 94912545.4
(22) Anmeldetag: 26.03.1994
(51) Int. Cl.: C12N 5/00, A61K 35/14

(54) **TUMORIZIDE T-LYMPHOZYTEN**
TUMORICIDE T LYMPHOCYTES
LYMPHOCITES T TUMORICIDES

(30) Priorität: 31.03.1993 DE 4310229
(43) Veröffentlichungstag der Anmeldung: 24.01.1996
(73) Patentinhaber: BOEHRINGER MANNHEIM GMBH, 68298 Mannheim (DE)
(72) Erfinder: JUNGFER, Herbert, D-82319 Starnberg (DE); BARCHET, Heinrich, D-82347 Bernried (DE); ALBERT, Winfried, D-82390 Eberfing (DE); WEIDLE, Ulrich, D-80336 München (DE)
(86) Internationale Anmeldenummer: EP9400960
(87) Internationale Veröffentlichungsnummer: WO9423014

(56) Entgegenhaltungen:
- EP-A- 0 203 403
- WO-A-90/10059
- DE-A- 3 923 848
- THE JOURNAL OF IMMUNOLOGY Bd. 137, Nr. 4 , 15. August 1986 , BALTIMORE MD, USA Seiten 1399 - 1406 D. THIELE ET AL. 'Leu-Leu-OMe sensitivity of human activated killer cells: Delineation of a distinct class of cytotoxic T lymphocytes capable of lysing tumor targets.' in der Anmeldung erwähnt
- THE JOURNAL OF EXPERIOMENTAL MEDICINE Bd. 160 , Juli 1984 , NEW YORK NY, USA Seiten 239 - 254 H. YSSEL ET AL. 'A cloned human T cell line cytotoxic for autologous and allogeneic B lymphoma cells.'

## Beschreibung

Gegenstand der Erfindung sind eine Säugerzellinie und deren aktive Fragmente, die bei einer Co-Kultivierung mit Lymphozyten, bei welcher man eine allogene Stimulierung vermeidet, Lymphozyten zu tumoriziden T-Zellen aktivieren, ein Verfahren zur Herstellung tumorizider T-Lymphozyten durch Co-Kultivierung von Lymphozyten mit solchen Zellinien oder mit deren aktiven Fragmenten, die durch dieses Verfahren erhältlichen tumoriziden T-Lymphozyten sowie die Verwendung dieser T-Lymphozyten zur Herstellung eines Therapeutikums, das Anwendung in der Tumortherapie findet.

Für die Eliminierung von krankhaft veränderten körpereigenen Zellen wie z.B. virusinfizierten Zellen oder Tumorzellen spielt die zelluläre Immunabwehr eine bedeutende Rolle. Dabei erkennen zytotoxisch wirkende T-Lymphozyten die veränderten körpereigenen Zellen anhand von Oberflächenantigenen. Diese Oberflächenantigene sind in der Regel Proteinfragmente, die von den Zellen gebildet werden und an der Zelloberfläche gebunden an Oberflächenrezeptoren des sogenannten Major-Histocompatibility-Komplex (MHC) vorliegen (Zinkernagel et al., Nature 248 (1974), 701 - 702 und Babbit et al., Nature 317 (1985), 359 - 361). Wenn sich diese Oberflächenantigene der Tumorzellen jedoch nur sehr geringfügig von den entsprechenden Antigenen gesunder Zellen unterscheiden, bildet das Immunsystem unter Umständen keine zytotoxisch wirkenden T-Lymphozyten, welche die Tumorzellen eliminieren könnten, aus.
Es wurden daher bereits Versuche unternommen, auch gegen solche Tumorzellen eine zelluläre Immunabwehr zu induzieren. Dazu wurde zunächst versucht, eine aktive Immunisierung mit unspezifischen Immunostimulantien wie dem Bacillus Calmette-Guérin (BCG), Corynebacterium parvum oder Vakzinen aus Tumorzellextrakten zu erreichen (Terry und Rosenberg (eds., Immunotherapy of Human Cancer (1982), Elsevier North Holland). Bessere Ergebnisse wurden mit dem Konzept der sogenannten adoptiven Immuntherapie erreicht. Hierbei werden Lymphozyten des Patienten in vitro aktiviert und dann reimplantiert. In den meisten Fällen erfolgt die in vitro Aktivierung zu solchen "promiscous killer cells " (D. Thiele et al., Immunology Today 10 (1989), 375 - 381) durch Zugabe von Interleukin-2. Die erhaltenen zytotoxischen Lymphozyten werden dann als Lymphokin-aktivierte Killer-Zellen (LAK-Zellen) bezeichnet (Rosenberg, Immunology Today 9 (1988), 58 - 62). Im Gegensatz zu zytotoxischen T-Lymphozyten sind LAK-Zellen für ihre Wirkung gegen Tumorzellen nicht auf eine korrekte Expression der MHC-Gene zur Erkennung der Tumorantigene angewiesen und im Gegensatz zu den Natural Killer Zellen des Immunsystems sind LAK-Zellen auch gegen frische Tumorzellen wirksam. Mit LAK-Zellen konnten auch bereits erste klinische Erfolge erzielt werden. Ein Nachteil dieser Form der adoptiven Immuntherapie sind jedoch Nebenwirkungen des über einen längeren Zeitraum in relativ hohen Dosierungen erforderlichen Interleukins-2. Dadurch kommt es vor allem zu einer Erhöhung der Permeabilität der Kapillaren und einer dadurch bedingten Organdysfunktion (Rosenberg, Immunology Today 9 (1988), 58 - 62, Rosenstein et al., Journal Immunology 137 (1986), 1735 - 1742 und Ettinghausen et al., Surg. Forum 37 (1987), 388 - 389). Zudem werden bei der Stimulierung mit Interleukin-2 auch solche LAK-Zellen erhalten, welche gegen gesunde körpereigene Zellen gerichtet sind (B. Chen et al., Cell. Immunol. 118 (1989), 458 - 469).

Auf der Suche nach effektiveren Verfahren zur adoptiven Immuntherapie wurden die zu aktivierenden Lymphozyten auch in Gegenwart von autologen Tumorzellen kultiviert (mixed lymphocyte tumor cultures, G. Fossati et al., International Journal of Cancer 42 (1988), 239 - 245; G. Degiovanni et al., Eur. J. Immunol. 18 (1988), 671 - 676; Wölfel et al., J. Exp. Med. 170 (1989), 797 - 810; Darrow et al., J. Immunol. 142 (1989), 3329 - 3335 und Notter et al., Int. J. Cancer 45 (1990), 834 - 841). Daneben wurde auch ein Verfahren zur Vermehrung von tumorinfiltrierenden Lymphozyten (TIL) in vitro beschrieben (Yron et al., J. Immunol. 125 (1980), 238 - 245). Im Gegensatz zu LAK-Zellen weisen diese tumorinfiltrierenden Lymphozyten eine hohe Tumorspezifität auf, d.h. sie sind nur wirksam gegen den Tumor, aus dem sie selbst isoliert wurden. Selbst gegen gleichartige Tumore eines anderen Patienten sind derartige tumorinfiltrierende Lymphozyten nicht wirksam. Dies grenzt ihre therapeutische Anwendbarkeit deutlich ein.

Aufgabe der Erfindung war es daher, tumorizide T-Lymphozyten zur Verfügung zu stellen, welche für eine Tumortherapie besser geeignet sind als die bislang bekannten in vitro aktivierten T-Lymphozyten.

Diese Aufgabe wird gelöst durch eine Säugerzellinie oder deren aktiven subzellulären Fraktionen, welche dadurch gekennzeichnet sind, daß
a) sie bei einer Co-Kultivierung mit Lymphozyten, bei der man eine allogene Stimulierung vermeidet, Lymphozyten zu tumoriziden T- Zellen aktivieren, ohne daß Mitogene oder Wachstumsfaktoren wie z.B. Interleukin-2 zugesetzt werden müssen, und
b) in ihrer Gegenwart die so aktivierten Lymphozyten ohne Zusatz von Interleukin-2 proliferieren.

Es hat sich überraschenderweise gezeigt, daß durch Co-Kultivierung mit einer erfindungsgemäßen Zellinie oder deren aktiven subzellulären Fraktionen aus Lymphozyten tumorizide T-Lymphozyten mit einer breiten tumoriziden Aktivität ohne HLA-Restriktion gewonnen werden können. Unter einer tumoriziden Aktivität ist dabei sowohl eine abtötende, insbesondere lysierende Wirkung auf die entsprechenden Tumorzellen sowie auch eine proliferationshemmende Wirkung auf diese Tumorzellen zu verstehen.

Unter einer Zellinie werden solche Zellen verstanden, welche eine solche Fähigkeit zur unbeschränkten Vermehrung aufweisen, wie sie für HeLa-Zellen (ATCC CCL 2) charakteristisch ist (James D. Watson et al., Molecular Biology of the Gene, 4th edition, The Benjamin/Cummings Publishing Co., Inc. (1987), S. 963). Derartige Zellinien werden z. B. durch Immortalisierung von humanen Blutlymphozyten erhalten. Die Immortalisierung erfolgt vorzugsweise durch Fusion mit Zytoplasten aus der Maus-Myelom-Zellinie Ag8.653 gemäß dem in EP-B 0 093 436 oder in EP-B 0 256 512 (deren Inhalt auch Gegenstand der vorliegenden Patentanmeldung ist) beschriebenen Verfahren. Die so erhaltenen immortalisierten Lymphozytenlinien werden dann mit humanen Spenderlymphozyten co-kultiviert.

Als Lymphozyten werden vorzugsweise Blut lymphozyten verwendet. Es ist jedoch auch möglich, tumorinfiltrierende Lymphozyten (TIL) sowie Lymphozyten aus Milz oder Lymphknoten zu verwenden. Dabei ist es bevorzugt, die Lymphozytenpräparation vor Anwendung zu reinigen. Bei Verwendung von Blutlymphozyten ist es zweckmäßig, insbesondere die Erythrozyten weitgehend abzureichern und die mononukleären Zellen anzureichern. Ebenso vorteilhaft ist es, die Zellen, die durch die erfindungsgemäße Zellinie oder deren aktive Fragmente allogen stimuliert werden können, abzureichern.

Zur Vermeidung einer allogenen Stimulierung werden vorzugsweise die einer solchen Stimulierung zugänglichen Lymphozyten aus der Spenderlymphozyten-Population vor der Co-Kultivierung eliminiert. Dazu werden Monozyten, Makrophagen, Natural Killer Zellen sowie MHC-restringierte zytotoxische T-Zellen, insbesondere auch solche gegen allogenes MHC der Aktivatorzellinie, und deren Vorläuferzellen vorzugsweise gemäß Thiele und Lipsky (The Journal of Immunology, Vol. 136, Nr. 3 (1986), S. 1038 - 1048) durch Inkubation mit L-Leucyl-L-Leucinmethylester eliminiert. Nach der Co-Kultivierung werden diejenigen immortalisierten Lymphozytenlinien ausgewählt, die bei dieser Co-Kultivierung eine Aktivierung der Spenderlymphozyten zu tumoriziden T-Lymphozyten bewirken. Dabei werden vorzugsweise solche aktivierenden Lymphozytenlinien weiter untersucht, die bei der Co-Kultivierung durch die von ihnen aktivierten Spenderlymphozyten lysiert werden. Zur weiteren Selektion werden diese aktivierenden Lymphozytenlinien zusammen mit den von ihnen aktivierten Spenderlymphozyten und einer Reihe verschiedener Tumorzellinien kultiviert. Schließlich werden diejenigen aktivierenden Lymphozytenlinien ausgewählt, die zu aktivierten Spenderlymphozyten mit tumorizider Wirkung gegen die untersuchten Tumorzellinien führen. Unter einer tumoriziden Wirkung ist dabei nicht nur die Abtötung insbesondere Lyse der untersuchten Tumorzellinien zu verstehen, sondern auch eine proliferationshemmende Wirkung. Diese tumorizide Wirkung kann z.B. über die dem Fachmann geläufigen Zytotoxizitätstests erkannt werden, z.B. daran, daß die morphologisch sowohl von den tumoriziden T-Lymphozyten als auch von der aktivierenden Lymphozytenlinie unterscheidbaren Tumorzellinien aus der Co-Kultur verschwinden oder zumindest bei längerer Kultivierung von den tumoriziden T-Lymphozyten überwachsen werden. Die tumoriziden T-Lymphozyten sind vorzugsweise CD3⁺, CD4⁺ und/oder CD8⁺.

Ein bevorzugter Gegenstand der Erfindung ist eine Lymphozyten-Zellinie, besonders bevorzugt eine B-Lymphozyten-Zellinie, oder deren aktive Fraktionen, welche dadurch gekennzeichnet sind, daß
a) sie bei einer Co-Kultivierung mit Lymphozyten, bei der man eine allogene Stimulierung vermeidet, Lymphozyten zu tumoriziden T-Zellen aktivieren, ohne daß Mitogene oder Wachstumsfaktoren wie z.B. Interleukin-2 zugesetzt werden müssen und
b) in ihrer Gegenwart die so aktivierten Lymphozyten ohne Zusatz von Interleukin-2 proliferieren.

Besonders bevorzugt sind die humanen B-Zellinien HB 654 und HB 617.

Mit den erfindungsgemäßen Zellinien und aktiven Fraktionen ist es möglich, durch einfache Co-Kultivierung mit Lymphozyten, bei der man vorzugsweise eine allogene Stimulierung vermeidet, eine Aktivierung dieser Lymphozyten zu tumoriziden T-Zellen zu bewirken. Diese Aktivierung erfolgt vorzugsweise unter direktem Kontakt der Zellinien oder deren aktiven Fraktionen (Fragmenten) mit den Lymphozyten. Es hat sich gezeigt, daß die Zugabe von Antikörper gegen IL2 und/oder den IL2-Rezeptor die aktivierende Wirkung der erfindungsgemäßen Zellinien inhibieren.

Ein weiterer Gegenstand der Erfindung ist daher ein Verfahren zur Herstellung tumorizider T-Lymphozyten durch Co-Kultivierung von Lymphozyten mit einer erfindungsgemäßen Zellinie oder deren aktiven Fragmenten.

Zur Durchführung des erfindungsgemäßen Verfahrens werden zunächst vorzugsweise aus dem Blut oder aus Tumoren eines Spenders Lymphozyten (vorzugsweise mononukleäre Lymphozyten) nach bekannten Verfahren z.B. über eine Ficoll-Dichtegradienten-Zentrifugation isoliert. Anschließend werden die übrig behaltenen Lymphozyten in üblichem Lymphozytenkulturmedium zusammen mit einer erfindungsgemäßen Zellinie oder deren aktiven Fragmenten, vorzugsweise mit den humanen B-Zellinien HB 654 und/oder HB 617 unter Bedingungen kultiviert, die einen Zellkontakt ermöglichen. Dabei wird die erfindungsgemäße Zellinie vorzugsweise in einem Unterschuß von etwa 1:100 zu den Lymphozyten gegeben. Die Kultivierung wird fortgesetzt, bis anhand der Eliminierung der aktivierenden Zellinie die Aktivierung tumorizider T-Zellen erkennbar wird. Hierfür ist in der Regel eine Kultivierung für etwa 8 Tage erforderlich. Durch die erfindungsgemäße Co-Kultivierung wird eine Aktivierung und Vermehrung tumorizider T-Lymphozyten erreicht, ohne daß Wachstumsfaktoren oder Mitogene wie z.B. Lymphokine, insbesondere Interleukin-2 zugegeben werden müssen. Dies ist für eine therapeutische Verwendung der erhaltenen tumoriziden T-Lymphozyten von besonderem Vorteil, da derartige Faktoren Nebenwirkungen bei der therapeutischen Anwendung auslösen können. Eine andauernde Proliferation der erhaltenen tumoriziden T-Lymphozyten erfordert jedoch eine ständige Gegenwart der erfindungsgemäßen Zellinie oder deren aktiven Fragmenten und die Möglichkeit von Zell-Zell-Kontakten. Da sich die tumorizide Aktivität der erhaltenen aktivierten Lymphozyten auch gegen die erfindungsgemäße Zellinie richtet, ist für eine andauernde Proliferation daher eine ständige Zufuhr dieser Zellinie erforderlich. Ohne eine solche Zugabe stagniert zwar die Proliferation der tumoriziden T-Lymphozyten nach ein bis zwei Tagen, die tumoriziden T-Lymphozyten überleben jedoch drei bis vier Wochen, wobei sie sich von Blasten in sehr kleine Zellen umwandeln, die sich zu Aggregaten zusammenschließen. Sie behalten dabei ihre tumorizide Aktivität und können durch Zugabe der erfindungsgemäßen Zellinie oder deren aktiven Fragmenten nach einer Latenzzeit von drei bis sechs Tagen wieder in einen proliferierenden Zustand überführt werden.

Zur Co-Kultivierung mit der erfindungsgemäßen Zellinie kann außer vitalen, vermehrungsfähigen Zellen auch eine mit Mitomycin behandelte, lethal bestrahlte oder chemisch, z.B. mit Formaldehyd, fixierte erfindungsgemäße Zellinie sowie eine subzelluläre Fraktion wie z.B. eine Membranfraktion, Membranvesikel oder ein Extrakt aus einer solchen subzellulären Fraktion verwendet werden. Weiterhin kann die erfindungsgemäße Zellinie auch mit anderen Zellen fusioniert und die erhaltenen Fusionszellen zur Aktivierung verwendet werden.

Ein weiterer Gegenstand der Erfindung ist daher ein Verfahren zur Herstellung tumorizider T-Lymphozyten durch Co-Kultivierung von Lymphozyten aus dem Blut, bei welcher man vorzugsweise eine allogene Stimulierung vermeidet, mit einer erfindungsgemäßen Zellinie, aktiven subzellulären Fraktionen der erfindungsgemäßen Zellinie, ohne daß Mitogene oder Wachstumsfaktoren wie Interleukin-2 zugesetzt werden.

Da bei einem derartigen Verfahren zur Herstellung zytotoxischer T-Lymphozyten keine Wachstumsfaktoren oder Mitogene zugesetzt werden müssen und die erhaltenen tumoriziden T-Lymphozyten auch zur weiteren Proliferation keinen Zusatz von Interleukin-2 benötigen, sind sie für eine Anwendung in der Tumortherapie besser geeignet als die bislang bekannten promiscous killer cells wie z.B. LAK-Zellen. Gegenüber den tumorinfiltrierenden Lymphozyten sind sie aufgrund ihrer breiteren tumoriziden Aktivität besser für einen therapeutischen Einsatz geeignet. Im Gegensatz zu Natural Killer Zellen sind die nach dem erfindungsgemäßen Verfahren hergestellten tumoriziden Zellen T-Zellen.

Ein weiterer Gegenstand der Erfindung sind daher tumorizide T-Lymphozyten mit einer breiten tumoriziden Aktivität, welche sich dadurch auszeichnen, daß
a) sie auf die Tumorzellinien MOLT-4, Jurkat, THP-1, HL-60, HeLa, K-562, Malme-3M und Y79 eine tumorizide Wirkung ausüben und
b) im Kulturüberstand dieser tumoriziden T-Lymphozyten während der Proliferation dieser Zellen in Gegenwart der Zellinie HB 654 oder HB 617 bei einer Nachweisgrenze von 0,5 IU/ml kein Interleukin-2 nachweisbar ist.

Die erfindungsgemäßen tumoriziden T-Lymphozyten sind also erhältlich durch einfache Co-Kultivierung von Lymphozyten mit einer erfindungsgemäßen Zellinie oder aktiven subzellulären Fraktionen dieser Zellinie oder einem Fusionsprodukt dieser Zellinie mit einer anderen Zelle, bis die Aktivierung von Lymphozyten zu tumoriziden T-Zellen erkennbar ist, z.B. anhand der Eliminierung der aktivierenden Zellinie. Es hat sich überraschenderweise gezeigt, daß die so erhaltenen tumoriziden T-Lymphozyten auf eine Vielzahl von Tumorzellinien wie z.B. MOLT-4, Jurkat, THP-1, HL-60, HeLa, K-562, Malme-3M und Y79 eine tumorizide Wirkung ausüben. Weiterhin zeichnen sich diese tumoriziden T-Lymphozyten dadurch aus, daß in ihrem Kulturüberstand weder während der Aktivierung noch bei der anschließenden Proliferation der aktivierten Zellen Interleukin-2 nachweisbar ist (IL2 ELISA; DuPont, Katalog Nr. NEK-057; untere Nachweisgrenze 0,5 IU/ml).

Wie bereits ausgeführt, sind unter Verwendung der erfindungsgemäßen aktivierenden Zellinie humane tumorizide T-Lymphozyten erhältlich, ohne daß Mitogene oder Wachstumsfaktoren wie z.B. Lymphokine, insbesondere Interleukin-2, zugesetzt werden müssen, die bei einer Therapie zu Nebenwirkungen führen können. Ein weiterer Gegenstand der Erfindung ist daher die Verwendung der erfindungsgemäßen tumoriziden T-Lymphozyten zur Herstellung eines Therapeutikums, das Anwendung in der Tumortherapie findet. Für eine solche therapeutische Verwendung werden die erfindungsgemäßen tumoriziden T-Lymphozyten gemäß dem Fachmann geläufigen Verfahren gewaschen (z. B. durch Zentrifugation und Resuspension des Pellets in physiologischer Kochsalzlösung bei mehrfacher, z. B. dreifacher Wiederholung), gegebenenfalls isoliert und in ein für die Applikation geeignetes Medium (z.B. physiologische Kochsalzlösung) aufgenommen.

Neben dieser ex vivo-Aktivierung von Lymphozyten zu tumoriziden T-Lymphozyten können Lymphozyten auch in vivo durch Applikation einer erfindungsgemäßen aktivierenden Zellinie oder subzellulären Fraktionen dieser Zellinie zu tumoriziden T-Lymphozyten aktiviert werden. Für eine solche therapeutische Verwendung wird die aktivierende Zellinie nach dem Fachmann geläufigen Verfahren gewaschen und in einem für die Applikation geeigneten Medium wie z.B. physiologischer Kochsalzlösung aufgenommen.

Ein weiterer Gegenstand der Erfindung ist daher die Verwendung einer erfindungsgemäßen aktivierenden Zellinie oder einer aktiven, die Induktion von Lymphozyten zu tumoriziden T-Zellen bewirkenden subzellulären Fraktion (Fragmente) oder einem entsprechenden Derivat dieser Zellinie zur Herstellung eines Therapeutikums, das Anwendung in der Tumortherapie findet.

Insbesondere die aktiven subzellulären Fraktionen eignen sich für eine direkte in vivo-Anwendung. Mit diesen Fraktionen können Lymphozyten im Körper direkt zu tumoriziden T-Lymphozyten aktiviert werden. Besonders vorteilhaft ist es, diese Fraktionen direkt am Tumor anzuwenden, so daß tumorinfiltrierende Lymphozyten zu tumoriziden T-Lymphozyten aktiviert werden.

Unter einer aktiven subzellulären Fraktion wird eine Fraktion einer erfindungsgemäßen Zellinie verstanden, die in analoger Weise die Induktion von Lymphozyten zu tumoriziden T-Zellen bewirkt, wie die erfindungsgemäßen Zellinien (z. B. HB 617 und HB 654). Derartige Fraktionen können beispielsweise subzelluläre Vesikel sein, die durch hypotonen Schock gewonnen werden, oder zellfreie Membranvesikel, die durch Inkubation mit Cytochalasin B gewonnen werden. Ebenso geeignet ist ein Eluat aus den erfindungsgemäßen Zellinien, das beispielsweise nach Inkubation mit Natriumchlorid und Natriumcitrat gewonnen werden kann. Derartige Fraktionen der erfindungsgemäßen Zellinien können nach den dem Fachmann geläufigen Methoden weiter auf gereinigt werden, beispielsweise durch chromatographische Reinigung, wobei nach jedem Reinigungsschritt die Aktivität, der Fraktion (die Eigenschaft der Bildung von tumoriziden T-Lymphozyten) überprüft werden muß.

Gegenstand der Erfindung ist demnach auch ein Verfahren zur Herstellung einer aktiven Fraktion aus Lymphozyten-Zellinien, die
a) bei einer Co-Kultivierung mit Lymphozyten, bei der man eine allogene Stimulierung vermeidet, Lymphozyten zu tumoriziden T-Zellen aktiviert, ohne daß Mitogene oder Wachstumsfaktoren zugesetzt werden müssen und
b) in ihrer Gegenwart die so aktivierten Lymphozyten ohne Zusatz von Interleukin 2 proliferieren,
dadurch gekennzeichnet, daß eine Säugerzellinie, die diese Eigenschaften besitzt,
(i) fraktioniert wird,
(ii) die Fraktionen getrennt werden und geprüft wird, ob diese Fraktionen in analoger Weise wie die Ausgangs zellinie Lymphozyten zu tumoriziden T-Zellen aktivierten,
(iii) eine solche aktive Fraktion ausgewählt und unter Überprüfung ihrer Aktivität bis zum gewünschten Reinheitsgrad weiter fraktioniert und isoliert wird.

Die tumoriziden T-Lymphozyten können ebenfalls zur Elimination von Tumorzellen in einer Zellpräparation ex vivo eingesetzt werden. Vorzugsweise können damit aus Stammzellisolaten (z. B. Knochenmarkstammzellen) durch Co-Kultivierung mit tumoriziden T-Lymphozyten oder einer erfindungsgemäßen Lymphozytenzellinie Tumorzellen eliminiert werden (purging). Die so gereinigten Stammzellen können beispielsweise nach einer Strahlen- oder Chemotherapie dem Patienten wieder implantiert werden (autologe Knochenmarkstransplantation).

Schließlich sind auch entsprechende therapeutische Zusammensetzungen ein Gegenstand dieser Erfindung, welche erfindungsgemäße tumorizide T-Lymphozyten bzw. eine erfindungsgemäße aktivierende Zellinie oder eine die Induktion von Lymphozyten zu tumoriziden T-Zellen bewirkende subzelluläre Fraktion jeweils zusammen mit den üblichen pharmazeutischen Träger-, Füll- und/oder Hilfsstoffen enthält.

Die erfindungsgemäße Zellinie HB 654 wurde am 24.03.1993 bei der Deutschen Sammlung für Zellkulturen und Mikroorganismen GmbH, Mascheroder Weg 1 b, D-3300 Braunschweig unter der Nummer DSM ACC 2122 hinterlegt.

Die erfindungsgemäße Zellinie HB 617 wurde am 11.03.94 bei der Deutschen Sammlung für Zellkulturen und Mikroorganismen GmbH, Mascheroder Weg 1 b, D-3300 Braunschweig unter der Nummer DSM ACC 2166 hinterlegt.

Die Erfindung wird durch die folgenden Beispiele und Abbildungen näher erläutert.
- Fig. 1: zeigt die Wirkung von erfindungsgemäßen Vesikeln auf die Anzahl der vitalen Blutzellen (PBL) und die Bildung von Lymphoblasten (d:Tage).
- Fig. 2: zeigt die BrdU-Freisetzung nach Stimulation mit erfindungsgemäßen Vesikeln als Maß für die Killeraktivität der gebildeten Lymphoblasten.

- Kurve 1:: Stimulation mit HB-Zellen
- Kurve 2:: Stimulation mit Eluat
- Kurve 3:: Spontanfreisetzung
- Kurve 4:: Leerwert

### Beispiel 1

### Herstellung einer Zellinie, die bei Co-Kultivierung mit Lymphozyten unter Vermeidung allogener Stimulierung zur Induktion von Lymphozyten zu tumoriziden T-Zellen führt

Die Herstellung einer erfindungsgemäßen Aktivatorzellinie erfolgt durch Immortalisierung von Lymphozyten gemäß dem in EP-B 0 093 436 beschriebenen Verfahren. Hierzu werden zunächst humane periphere Blutlymphozyten über eine Ficoll-Gradienten-Zentrifugation isoliert. Zytoplasten aus der Maus-Myelom-Zellinie P3X63 Ag8.653 (ATCC CRL1580) werden wie in EP-B 0 093 436 beschrieben durch Behandlung mit Cytochalasin B hergestellt. Jeweils 1 x 10⁷ der humanen peripheren Blutlymphozyten werden mit 1 x 10⁷ Zytoplasten der Myelomzellinie Ag8.653 vermischt und durch Zentrifugieren sedimentiert. Die überstehende Flüssigkeit wird sorgfältig entfernt. Zu dem Sediment werden 0,8 ml 50%iger Polyethylenglycol-4000-Lösung bei 37°C unter ständigem sanften Schütteln über einen Zeitraum von 1 min. gleichmäßig langsam zugegeben. Anschließend erfolgt die Zugabe von 5 ml Dulbecco's minimal essential medium (DMEM) bei Raumtemperatur über einen Zeitraum von 5 min. Nach Zugabe von weiteren 20 ml DMEM werden die Zellen sedimentiert, in 5 ml frischem DMEM-Vollmedium resuspendiert und in die Vertiefungen einer mit murinen Bauchhöhlen-Makrophagen als "feeder cells" beschichteten Zellkulturplatte verteilt. Die Einzelkulturen werden dann im Abstand von zwei bis drei Tagen mit DMEM-Vollmedium gefüttert. Schließlich wird der Klon selektioniert, welcher Lymphozyten zu tumoriziden T-Lymphozyten aktivieren kann. Dazu werden periphere Blutlymphozyten zur Eliminierung von Zellen, die über eine allogene Stimulierung zu tumoriziden T-Zellen aktiviert werden können, zunächst wie in Beispiel 2 beschrieben mit L-Leucyl-L-Leucinmethylester inkubiert. Die so erhaltene Lymphozyten-Population wird dann mit der zu testenden immortalisierten Lymphozytenlinie unter Bedingungen co-kultiviert, die einen direkten Zell-Zell-Kontakt ermöglichen. Solche immortalisierten Lymphozytenlinien, die bei einer solchen Co-Kultivierung durch die von ihnen aktivierten tumoriziden T-Lymphozyten lysiert werden, werden weiter untersucht. Dazu werden diese ausgewählten Zellinien zusammen mit den von ihnen aktivierten tumorigenen T-Lymphozyten sowie verschieden Tumorzellinien kultiviert. Schließlich werden solche immortalisierten aktivierenden Lymphozytenlinien ausgewählt, die bei diesem Verfahren T-Lymphozyten mit tumorizider Wirkung auf verschiedene Tumorzellinien erzeugen. Die tumorizide Wirkung wird dabei dadurch erkannt, daß die sowohl von den tumoriziden T-Lymphozyten als auch von den aktivierenden Lymphozytenlinien morphologisch unterscheidbaren Tumorzellinien während dieser Co-Kultivierung aus der Kultur verschwinden oder zumindest im Vergleich zu einer unbehandelten Kontrollkultur in ihrer Anzahl abnehmen. Auf diese Weise wurde die Zellinie HB 654 erhalten.

In gleicher Vorgehensweise werden permanente, humane B-Lymphozytenlinien, die durch Infektion mit Epstein-Barr-Virus immortalisiert worden sind, auf Aktivator-Eigenschaften untersucht. Getestet werden 20 verschiedene, durch Einzel-Zell-Aufzucht klonierte, EBV-positive B-Linien in Cokultur mit Blutlymphozyten, die mit Leucyl-Leucin-Methylester vorbehandelt sind. Die Einsaatdichte der Blutlymphozyten beträgt 2 x 106 pro ml Kultur-medium (Iscove mod. DMEM + 15% FKS; BM). In getrennten Ansätzen werden von jeder der B-Linien 50-, 100- und 200-fach weniger Zellen zu den Blutlymphozyten zugemischt und bei 37°C in 5 % CO₂ Atmosphäre inkubiert. Auf diese Weise wurde die Zellinie HB 617 erhalten, die ebenfalls eine erfindungsgemäße Wirkung zeigt.

### Beispiel 2

### Herstellung tumorizider T-Lymphozyten durch Co-Kultivierung mit der humanen B-Zellinie HB 654

Aus humanem peripheren Blut werden die mononukleären Zellen in üblicher Weise durch Ficoll®-Gradienten-Zentrifugation gewonnen. Zur Eliminierung von Monoyzten, Makrophagen, Natural Killer Zellen sowie MHC restringierten zytotoxischen T-Zellen, insbesondere solchen gegen allogenes MHC der aktivierenden Zellinie HB 654, und deren Vorläufern werden die erhaltenen mononukleären Zellen in PBS für 15 min. bei Raumtemperatur mit 250 µmol/1 L-Leucyl-L-Leucinmethylester gemäß Thiele und Lipsky (The Journal of Immunology, Vol. 136, Nr. 3 (1986), S. 1038 - 1048) inkubiert. Anschließend werden die Zellen in Iscoves modifiziertes Dulbecco-Medium mit 15 % FKS aufgenommen und nach Zugabe von HB-654-Zellen im Unterschuß (ca. 1:100) für 6 bis 8 Tage bei 37°C inkubiert, bis tumorizide T-Lymphozyten an ihrer die Aktivatorzellinie eliminierenden Aktivität erkannt werden können.

### Beispiel 3

### Wirkung tumorizider T-Lymphozyten

Die gemäß Beispiel 2 erhaltenen tumoriziden T-Lymphozyten aus jeweils 20 verschiedenen Spendern werden zu Kulturen von humanen Tumorlinien (s. Tabellen I und II) gegeben. Die tumorizide Wirkung auf diese Tumorzellen wird unter dem Mikroskop verfolgt. Mit den erfindungsgemäßen tumoriziden T-Lymphozyten werden diese verschiedenen Tumorzellinien in ihrem Wachstum gehemmt oder abgetötet.

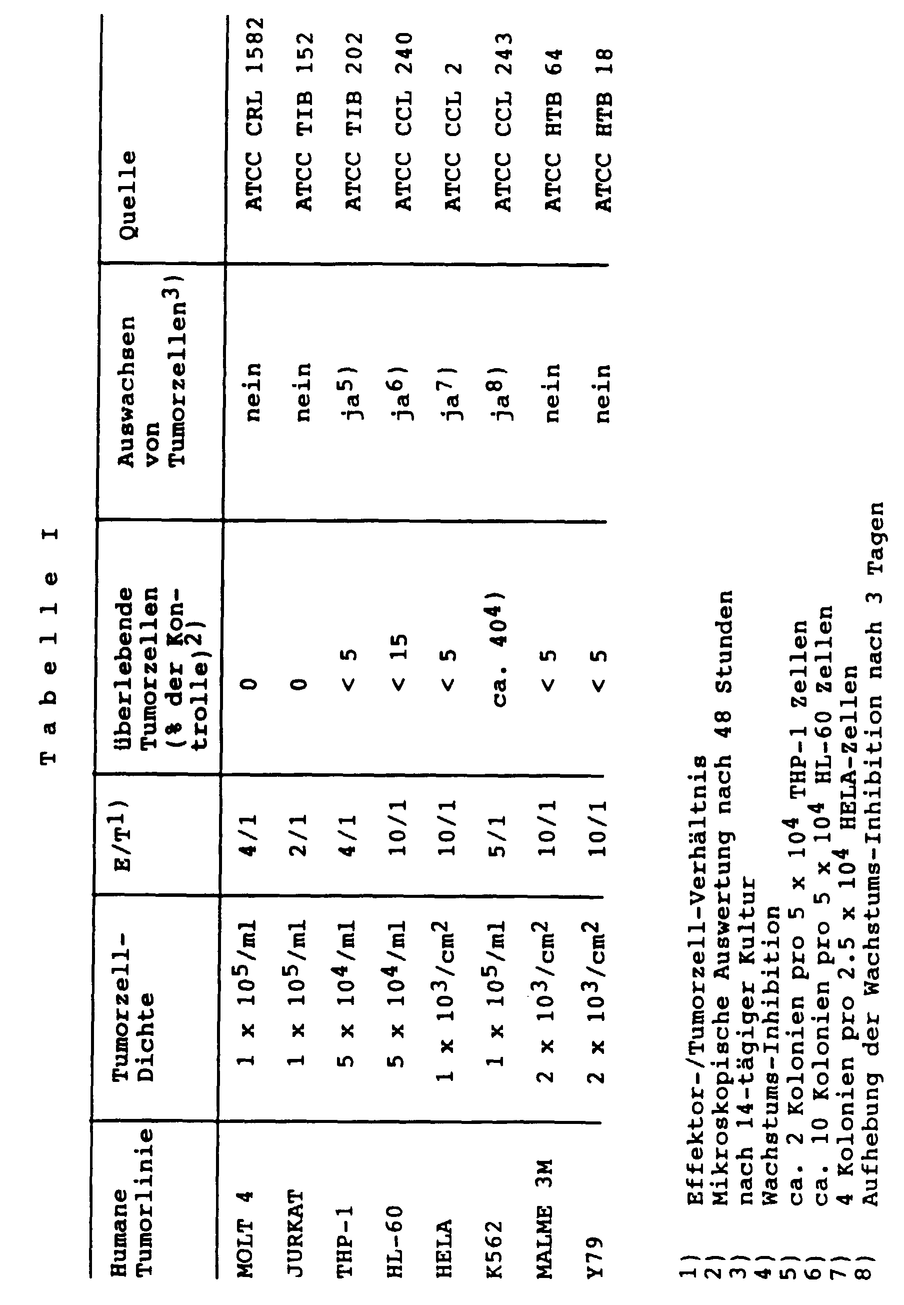

**Tabelle II**

| Humane Tumorlinie | Tumor-Typ |
|---|---|
| MOLT 4 | akute lymphoblastische Leukämie |
| | |
| Jurkat | akute T-Zell-Leukämie |
| | |
| THP-1 | akute Monozyten-Leukämie |
| | |
| HL-60 | Promyelozyten-Leukämie |
| | |
| HeLa | Cervix-Karzinom |
| | |
| K-562 | chronische myelogene Leukämie |
| | |
| Malme-3M | malignes Melanom |
| | |
| Y79 | Retinoblastom |

### Beispiel 4:

### Erzeugung von tumoriziden T-Lymphozyten (Killer-T-Zellen) mittels zellfreier Membran-Vesikel, die aus HB654-Zellen durch Behandlung mit Cytochalasin B hergestellt werden.

### A. Herstellung von Membranvesikeln:

Nach der von MAUL, G.D. et al (in: Techniques in Somatic Cell Genetics, Ed. J.W. SHAY; Plenum Press, New York, 1982) beschriebenen Methode werden auch HB654-Zellen durch Inkubation mit Cytochalasin B (CB, Aldrich Biochemicals) veranlaßt, Membranvesikel ("blebs") abzuschnüren, die durch Einwirkenlassen von Scherkräften von den Zellen (ohne Zellzerstörung) abgetrennt werden können.

HB654-Zellen aus Kulturen in logarithmischer Wachstumsphase werden zweimal in serumfreiem Kulturmedium (RPMI 1640, BM) gewaschen, in diesem in einer Dichte von ca. 2 x 10⁷ Zellen/ml suspendiert und auf 37°C erwärmt. CB (Stammlösung: 5 mg/ml DMSO) wird zugegeben (Endkonzentration: 25 µg/ml). Die Suspension wird 1 Minute bei 37°C inkubiert und sodann 1 Minute lang auf einer Vortex-Apparatur in Rotation gebracht. Durch niedrigtourige Zentrifugation werden die Zellen sedimentiert. Der vesikelhaltige Überstand wird durch ein 5 µm- Filter filtriert. An Ausbeute werden durchschnittlich 3 Membranvesikel von einer HB654-Zelle erhalten.

### B. Erzeugung von tumoriziden Killer-T-Zellen aus Blutlymphozyten:

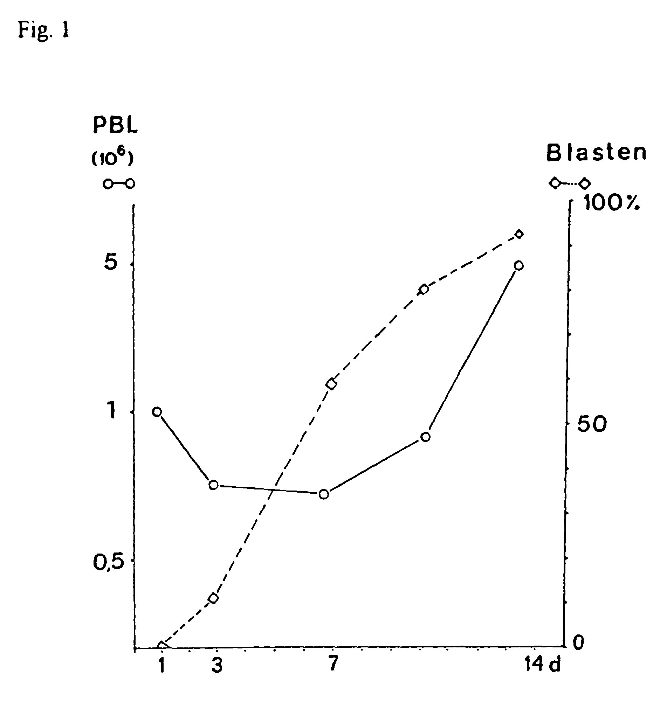

Mononukleäre Blutzellen werden, wie im Beispiel 2 beschrieben, durch Gradienten-Zentrifugation isoliert, mit Leucyl-Leucin-Methylester behandelt und in einer Dichte von ca. 2x 10⁶ Zellen pro ml eines Kulturmediums (Iscove mod. DMEM plus 15% FKS), dem pro ml ca. 2 x 10⁶ Vesikel zugemischt worden sind, inkubiert. Am Tag 7 und 10 werden die Zellen mit frischem Kulturmedium versorgt, das wiederum 2 x 10⁶ Vesikel pro ml enthält. Am 3, 7, 10 und 13 wird die Gesamtzahl der vitalen Blutzellen (PBL) und der Anteil an Lymphoblasten in der Kultur bestimmt (Fig.1). Die Bestimmung ergibt, daß die Zahl der lebenden Blutzellen zunächst abnimmt und am Tag 7 wiederum zunimmt. Am Tage 13 beträgt die Zahl an vitalen Zellen etwa das 5-fache der ursprünglich eingesäten Zellen. Der Anteil an Lymphoblasten steigt von 0% am Tag 1 der Kultur auf ca. 95% am Tag 13.

### C. Wirkung der mit Vesikeln induzierten Lymphoblasten auf Tumor-Zellen:

Die tumorizide Funktion der gemäß B. erhaltenen Lymphoblasten werden am Tag 14 nach Anlage der Kultur gegen die Tumorlinien Jurkat, THP-1 und HB654 geprüft. Die Zerstörung der Tumorzellen wird mit dem "Cellular DNA fragmentation ELISAkit" (Boehringer Mannheim GmbH, DE, Best.Nr. 1585045) nach Vorschrift des Herstellers gemessen.

Prinzip: Tumor(Ziel-)zellen werden metabolisch markiert durch Zugabe von 5-Brom-2'deoxy-Uridin (BrdU) in das Kulturmedium. BrdU wird anstelle von Thymidin von proliferierenden Zellen in die DNA eingebaut. Zytotoxische Effekte auf diese Zielzellen können sodann anhand von Freisetzung von BrdU-markierter DNA gemessen werden, und zwar mittels eines ELISA (enzyme-linked immunosorbent assay), bei dem wandgebundene Anti-DNA-Antikörper und Anti-BrdU-Antikörper-Peroxidase-Konjugat verwendet werden.

In allen 3 Tumorlinien wird nach 24-stündiger Cokultur mit den Lymphoblasten gemäß B. bei einem Effektor/Target-Verhältnis von 4/1 mindestens 80% und bei einem E/T-Verhältnis von 10/1 100% der maximal freisetzbaren Tumorzell-DNA im Kulturüberstand gefunden.

### Beispiel 5:

### Erzeugung von Killer-T-Zellen durch subzelluläre Vesikel, welche auf physikalische Weise, durch hypotonen Schock, gewonnen wurden.

Als eine weitere Methode, um subzelluläre Fragmente zu gewinnen, die geeignet sind, den beanspruchten Effekt zu erzeugen, wurde eine nach Jett et al. modifizierten Methode verwendet (Jett et al., J.Biol.Chem. 252 (1977), 2134-2142). Zur Gewinnung der Vesikel wurde wie folgt vorgegangen:

Zellen der Stimulator-Zellinie HB 654 wurden in Earls-Puffer (enthaltend 0,9 mM Calciumchlorid und 0,5 mM Magnesiumchlorid im PBS-Puffer) gewaschen und anschließend in dem gleichen Puffer in 1 % des ursprünglichen Kulturvolumens aufgenommen. Zur Erzeugung der Vesikel wurde 90 %iges Glycerin zu einer Endkonzentration von 30 % dazu gegeben, und zwar in drei Schritten in 5 min Intervallen. Die mit Glycerin beladenen Zellen wurden zentrifugiert (1200 x g fr 10 min, 4°C) und der Überstand verworfen. Zum Zellsediment wurde Lysepuffer (ca. 1 % des ursprünglichen Kulturvolumens; 10 mM Tris/HCl , pH 7,4, 1mM MgCl₂, 1 mM CaCl₂) mit rascher Durchmischung gegeben und 5 min im Eiswasser inkubiert. Anschließend erfolgten mehrere Zentrifugationsschritte, mit denen Zelltrümmer abgereichert und die Vesikelfraktion angereichert wurden. Eine erste Zentrifugation erfolgt mit 700 x g für 10 min. Der Überstand wurde einer zweiten Zentrifugation von 700 x g für 10 min unterworfen, der Niederschlag wurde verworfen. Der Niederschlag der zweiten Zentrifugation wurde ebenfalls verworfen und die Zentrifugation erneut durchgeführt. Wieder wurde der Niederschlag verworfen und der verbleibende Überstand einer Zentrifugation mit 2300 x g für 10 min unterworfen. Hier wurde der Überstand verwendet, um in einer letzten Zentrifugation von 4500 x g für 10 min die Vesikel in den Niederschlag zu verbringen. Der aufgenommene Niederschlag wurde noch einmal durch einen 5 µm-Filter gefiltert und anschließend eingesetzt. Um die stimulierende Eigenschaft der so gewonnenen Vesikel zu überprüfen, wurden diese in einem Stimulationsansatz, der wie vorher beschrieben durchgeführt wurde, eingesetzt. Dazu wurden aus 1 x 10⁸ HB 654-Zellen, wie oben beschrieben, Vesikel gewonnen, anschließend wurden diese zur Prägung von peripheren Blutlymphozyten (2 x 10⁷-Zellen, nach Behandlung mit Leucyl-Leucin-Methylester) eingesetzt. Dabei wurde genau so vorgegangen wie in den Beispielen 2 und 3 beschrieben.

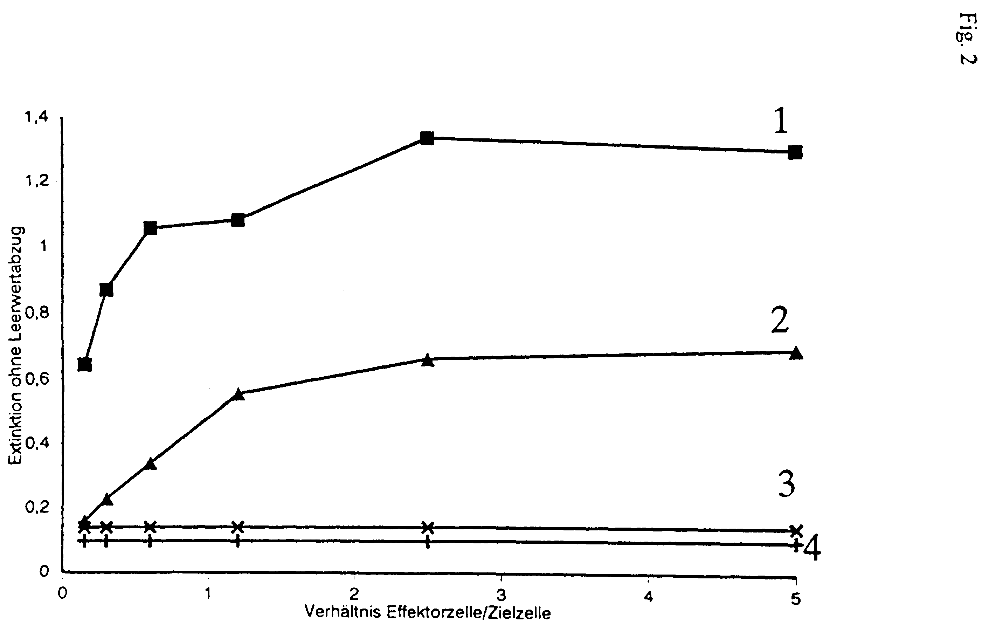

Zur Auswertung der Ergebnisse wurde ein Test auf "Killaktivität" durchgeführt. Dieser Test wurde, wie in Beispiel 3 beschrieben, durchgeführt. Dabei wurden die nach obigem Schema erzeugten Killer-T-Zellen als Effektor-Zellen; die T-Zell-Tumorlinie MOLT-4 als Zielzelle verwendet. Zur Quantifizierung der Lyserate der Zielzellen wurde dabei der "Cellular DNA Fragmentation ELISA-Kit" von Boehringer Mannheim GmbH verwendet. Es wurde vorgegangen wie in Beispiel 4 angegeben. Das Ergebnis des Testes ist in Fig. 2 wiedergegeben. Es zeigte sich, daß die durch Vesikel gebildeten Blasten Killer-T-zellaktivität besaßen.

Der in Fig. 2 beschriebene Leerwert entspricht einer Extinktion des Reagenses ohne Zusatz von Zellen. Der Meßwert für Spontanfreisetzung entspricht einer Extinktion, die bei Zugabe der Zielzellen ohne Effektorzellen erhalten wurde.

### Beispiel 6:

### Erzeugung von Killer-T-Zellen durch ein Eluat aus der Stimulatorzellinie, welches durch Inkubation mit einem geeigneten Puffer erzeugt wird.

Als eine weitere Methode, um subzelluläre Fragmente zu gewinnen, die geeignet sind, den beanspruchten Effekt zu erzeugen, wurde eine im folgenden beschriebene Methode verwendet:

Zellen der Stimulator-Zellinie HB 654, die nach dem in den anderen Beispielen genannten Methoden angezogen worden waren, wurden dreimal in Hanks Balanced Salt Solution (HB SS, Boehringer Mannheim GmbH, DE) gewaschen und anschließend in 150 mmol/l NaCl, 15 mmol/l Na-citrat, pH 7,2, in einer Dichte von etwa 2 x 10⁷ Zellen/ml aufgenommen. Anschließend erfolgte eine Inkubation für 30 min bei 37°C. Danach wurden die Zellen durch eine Zentrifugation für 7 min bei 4.500xg abzentrifugiert.
Der so gewonnene Überstand stellte das Eluat dar. Der Überstand wurde noch einmal durch einen 5 µm-Filter gefiltert und anschließend eingesetzt. Um die stimulierende Eigenschaft des so gewonnenen Eluates zu überprüfen, wurde dieses in einem Stimulationsansatz, der wie vorher beschrieben durchgeführt wurde, eingesetzt. Dazu wurden aus 2 x 10⁷ HB 654-Zellen, wie oben beschrieben, Eluat gewonnen, anschließend wurde dieser zur Prägung von peripheren Blutlymphozyten (2 x 10⁷-Zellen) eingesetzt. Dabei wurde genau so vorgegangen wie im vorherigen Beispiel beschrieben.

Zur Auswertung der Ergebnisse wurde ein Test auf "Killaktivität" durchgeführt. Dieser Test wurde, wie in den vorangegangenen Beispielen beschrieben, durchgeführt. Dabei wurden die nach obigem Schema erzeugten Killer-T-Zellen als Effektor-Zellen; Jurkat als Zielzelle verwendet. Zur Quantifizierung der Lyserate der Zielzellen wurde dabei der "Cellular DNA Fragmentation ELISA-Kit" von Boehringer Mannheim GmbH verwendet. Es wurde vorgegangen wie in Beispiel 4 angegeben. Es zeigte sich, daß die durch Eluat gebildeten Blasten Killer-T-Zellaktivität besaßen. Mit vier verschiedenen Vesikelpräparationen wurden als Extinktion für die BrdU-Freisetzung (nach Abzug der Extinktion für die Spontanfreisetzung) 0,121, 0,214, 0,269 und 0,114 erhalten.

## Patentansprüche

1. Säugerzellinie oder deren aktive Fraktionen, welche dadurch gekennzeichnet sind, daß
a) sie bei einer Co-Kultivierung mit Lymphozyten, bei der man eine allogene Stimulierung vermeiden, Lymphozyten zu tumoriziden T-Zellen aktivieren, ohne daß Mitogene oder Wachstumsfaktoren zugesetzt werden müssen, und
b) in ihrer Gegenwart die so aktivierten Lymphozyten ohne Zusatz von Interleukin-2 proliferieren.

2. Zellinie oder Fraktionen gemäß Anspruch 1, dadurch gekennzeichnet, daß es sich um eine Lymphozyten-Zellinie oder Fraktionen davon handelt.

3. Zellinien DSM ACC 2122 (HB 654) und DSM ACC 2166 (HB 617).

4. Verfahren zur Herstellung tumorizider T-Lymphozyten, dadurch gekennzeichnet, daß Lymphozyten mit einer Zellinie oder einer aktiven Fraktion dieser Zellinie gemäß einem der Ansprüche 1 bis 3 co-kultiviert werden, wobei man eine allogene Stimulierung vermeidet.

5. Verfahren gemäß Anspruch 4, dadurch gekennzeichnet, daß die Co-Kultivierung mit subzellulären aktiven Fraktionen der Zellinie gemäß einem der Ansprüche 1 bis 3 durchgeführt wird.

6. Tumorizide T-Lymphozyten, welche dadurch gekennzeichnet sind, daß
a) sie auf die Tumorzellinien ATCC CRL 1582 (MOLT-4), ATCC TIB 152 (Jurkat), ATCC TIB 202 (THP-1), ATCC CCL 240 (HL-60), ATCC CCL 2 (Hela), ATCC CCL 243 (K-562), ATCC HTB 64 (Malme-3M) und ATCC HTB 18 (Y 79) eine tumorizide Wirkung ausüben und
b) im Kulturüberstand dieser tumoriziden T-Lymphozyten während der Proliferation dieser Zellen in Gegenwart der Zellinie DSM ACC 2122 (HB 654) oder DSM ACC 2166 (HB 617) bei einer Nachweisgrenze von 0,5 IU/ml kein Interleukin-2 nachweisbar ist.

7. Verwendung humaner tumorizider T-Lymphozyten gemäß Anspruch 6 zur Herstellung eines Therapeutikums, das Anwendung in der Tumortherapie findet.

8. Verwendung einer Zellinie gemäß einem der Ansprüche 1 bis 3 oder einer die Induktion von Lymphozyten zu tumoriziden T-Zellen bewirkenden aktiven subzellulären Fraktion zur Herstellung eines Therapeutikums, das Anwendung in der Tumortherapie findet.

9. Therapeutische Zusammensetzung enthaltend tumorzide T-Lymphozyten gemäß Anspruch 6 sowie gegebenenfalls eine der üblicherweise verwendeten Träger-, Hilfs- oder Füllstoffe.

10. Therapeutische Zusammensetzung enthaltend eine Zellinie gemäß einem der Ansprüche 1 bis 3 oder eine die Induktion von Lymphozyten zu tumoriziden T-Zellen bewirkende aktive subzelluläre Fraktion, gegebenenfalls zusammen mit einem der üblicherweise verwendeten Träger-, Hilfs- oder Füllstoffe.

11. Verfahren zur Eliminierung der tumorigenen Wirkung von Tumorzellen in einer Zellpräparation, dadurch gekennzeichnet, daß die Zellpräparation mit tumoriziden T-Lymphozyten, die
a) sie auf die Tumorzellinien ATCC CRL 1582 (MOLT-4), ATCC TIB 152 (Jurkat), ATCC TIB 202 (THP-1), ATCC CCL 240 (HL-60), ATCC CCL 2 (Hela), ATCC CCL 243 (K-562), ATCC HTB 64 (Malme-3M) und ATCC HTB 18 (Y 79) eine tumorizide Wirkung ausüben und
b) im Kulturüberstand dieser tumoriziden T-Lymphozyten während der Proliferation dieser Zellen in Gegenwart der Zellinie DSM ACC 2122 (HB 654) bei einer Nachweisgrenze von 0,5 IU/ml kein Interleukin-2 nachweisbar ist,
inkubiert wird.

12. Verfahren nach Anspruch 11, dadurch gekennzeichnet, daß als Zellpräparation eine Stammzellenpräparation verwendet wird.

13. Verfahren zur Herstellung einer aktiven Fraktion aus Lymphozyten-Zellinien, die
a) bei einer Co-Kultivierung mit Lymphozyten, bei der man eine allogene Stimulierung vermeidet, Lymphozyten zu tumoriziden T-Zellen aktiviert, ohne daß Mitogene oder Wachstumsfaktoren zugesetzt werden müssen und
b) in ihrer Gegenwart die so aktiverten Lymphozyten ohne Zusatz von Interleukin 2 proliferieren,
dadurch gekennzeichnet, daß eine Säugerzellinie, die diese Eigenschaften besitzt,
(i) fraktioniert wird,
(ii) die Fraktionen getrennt werden und geprüft wird, ob diese Fraktionen in analoger Weise wie die Ausgangszellinie Lymphozyten zu tumoriziden T-Zellen aktivierten,
(iii) eine solche aktive Fraktion ausgewählt und unter Überprüfung ihrer Aktivität bis zum gewünschten Reinheitsgrad weiter fraktioniert und isoliert wird.

14. Verfahren nach Anspruch 13, dadurch gekennzeichnet, daß als Säugerzellinie DSM ACC 2122 (HB 654) oder DSM ACC 2166 (HB 617) verwendet wird.

15. Verfahren nach Anspruch 13 oder 14, dadurch gekennzeichnet, daß als aktive Fraktion ein Eluat, Membranvesikel oder subzelluläre Vesikel isoliert werden.

## Claims

1. Mammalian cell line or active fractions thereof, wherein
a) when they are co-cultured with lymphocytes during which allogenic stimulation is avoided, they activate lymphocytes to form tumoricidal T cells without having to add mitogens or growth factors and
b) the lymphocytes activated in this way proliferate in their presence without addition of interleukin 2.

2. Cell line or fractions as claimed in claim 1, wherein it is a lymphocyte cell line or fractions thereof.

3. Cell lines DSM ACC 2122 (HB 654) and DSM ACC 2166 (HB 617).

4. Process for the production of tumoricidal T lymphocytes, wherein lymphocytes are co-cultured with a cell line or an active fraction of this cell line as claimed in one of the claims 1 to 3 during which allogenic stimulation is avoided.

5. Process as claimed in claim 4, wherein the co-culture is carried out with subcellular active fractions of the cell line as claimed in one of the claims 1 to 3.

6. Tumoricidal T lymphocytes, wherein
a) they have a tumoricidal effect on the tumour cell lines ATCC CRL 1582 (MOLT-4), ATCC TIB 152 (Jurkat), ATCC TIB 202 (THP-1), ATCC CCL 240 (HL-60), ATCC CCL 2 (HeLa), ATCC CCL 243 (K-562), ATCC HTB 64 (Malme-3M) and ATCC HTB 18 (Y79) and
b) interleukin 2 is not detectable at a detection limit of 0.5 IU/ml in the culture supernatant of these tumoricidal T lymphocytes during proliferation of these cells in the presence of the cell line DSM ACC 2122 (HB 654) or DSM ACC 2166 (HB 617).

7. Use of human tumoricidal T lymphocytes as claimed in claim 6 for the production of a therapeutic agent which can be used in tumour therapy.

8. Use of a cell line as claimed in one of the claims 1 to 3 or an active subcellular fraction that induces lymphocytes to form tumoricidal T cells for the production of a therapeutic agent which can be used in tumour therapy.

9. Therapeutic composition containing tumoricidal T lymphocytes as claimed in claim 6 as well as optionally one of the carrier, auxiliary or filling agents usually used.

10. Therapeutic composition containing a cell line as claimed in one of the claims 1 to 3 or an active subcellular fraction that induces lymphocytes to form tumoricidal T cells together with optionally one of the carrier, auxiliary or filling substances usually used.

11. Process for the elimination of the tumorigenic action of tumour cells in a cell preparation, wherein the cell preparation is incubated with tumoricidal T lymphocytes which
a) have a tumoricidal effect on the tumour cell lines ATCC CRL 1582 (MOLT-4), ATCC TIB 152 (Jurkat), ATCC TIB 202 (THP-1), ATCC CCL 240 (HL-60), ATCC CCL 2 (HeLa), ATCC CCL 243 (K-562), ATCC HTB 64 (Malme-3M) and ATCC HTB 18 (Y79) and
b) no interleukin 2 is detectable in the culture supernatant of these tumoricidal T lymphocytes at a detection limit of 0.5 IU/ml during the proliferation of these cells in the presence of the cell line DSM ACC 2122 (HB 654).

12. Process as claimed in claim 11, wherein a stem cell preparation is used as the cell preparation.

13. Process for the production of an active fraction from lymphocyte cell lines which
a) activates lymphocytes to tumoricidal T cells in a co-culture with lymphocytes in which an allogenic stimulation is avoided without having to add mitogens or growth factors and
b) the lymphocytes activated in this way proliferate in their presence without addition of interleukin 2,
wherein a mammalian cell line which has these properties
(i) is fractionated,
(ii) the fractions are separated and it is examined whether these fractions activate lymphocytes to form tumoricidal T cells in an analogous manner to the initial cell line,
(iii) such an active fraction is selected and is fractionated further until the desired degree of purity is achieved while checking its activity and isolated.

14. Process as claimed in claim 13, wherein DSM ACC 2122 (HB 654) or DSM ACC 2166 (HB 617) is used as the mammalian cell line.

15. Process as claimed in claim 13 or 14, wherein an eluate, membrane vesicles or subcellular vesicles are isolated as the active fraction.

## Revendications

1. Lignées cellulaires de mammifère ou leurs fractions actives, caractérisées par le fait que
a) dans une co-culture avec des lymphocytes, dans laquelle on évite une stimulation allogène, elles activent les lymphocytes pour les transformer en lymphocytes T tumoricides, sans qu'il soit nécessaire d'ajouter des mitogènes ou des facteurs de croissance, et
b) en leur présence, les lymphocytes ainsi activés se multiplient sans addition d'interleukine 2.

2. Lignée cellulaire ou ses fractions selon la revendication 1, caractérisée par le fait qu'il s'agit d'une lignée cellulaire de lymphocytes ou de fractions de celle-ci.

3. Lignées cellulaires DSM ACC 2122 (HB 654) et DSM ACC 2166 (HB 617).

4. Procédé de préparation de lymphocytes T tumoricides, caractérisé par le fait que l'on co-cultive des lymphocytes avec une lignée cellulaire ou une fraction active de celle-ci selon l'une quelconque des revendications 1 à 3, en évitant une stimulation allogène.

5. Procédé selon la revendication 4, caractérisé par le fait que la co-culture est réalisée avec des fractions actives infracellulaires de la lignée cellulaire selon l'une quelconque des revendications 1 à 3.

6. Lymphocytes T tumoricides, caractérisés par le fait que
a) elles ont une action tumoricide vis-à-vis des lignées cellulaires ATCC CRL 1582 (MOLT-4), ATCC TIB 152 (Jurkat), ATCC TIB 202 (THP-1), ATCC CCL240 (HL-60), ATCC CCL2 (Hela), ATCC CCL 243 (K-562), ATCC HTB 64 (Malme-3M) et ATCC HTB 18 (Y 79), et
b) dans le surnageant de culture de ces lymphocytes T tumoricides, pendant la multiplication de ces cellules en présence de la lignée cellulaire DSM ACC 2122 (HB 654) ou DSM ACC 2166 (HB 617), pour une limite de détection de 0,5 UI/ml, des interleukine 2 ne sont pas détectables.

7. Utilisation de lymphocytes T tumoricides humains selon la revendication 6 pour la préparation d'un médicament qui est utilisable dans le traitement de tumeurs.

8. Utilisation d'une lignée cellulaire selon l'une quelconque des revendications 1 à 3 ou d'un fraction infracellulaire active, provoquant la transformation des lymphocytes en lymphocytes T tumoricides, pour la préparation d'un médicament qui est utilisable dans le traitement de tumeurs.

9. Composition thérapeutique contenant des lymphocytes T tumoricides selon la revendication 6 ainsi qu'éventuellement un véhicule, adjuvant ou charge usuel.

10. Composition thérapeutique contenant une lignée cellulaire selon l'une quelconque des revendications 1 à 3 ou une fraction infracellulaire active, provoquant la transformation des lymphocytes en lymphocytes T tumoricides, éventuellement avec un véhicule, adjuvant ou charge usuel.

11. Procédé d'élimination de l'action tumorigène de cellules tumorales dans une préparation cellulaire, caractérisé par le fait que la préparation cellulaire est incubée avec des lymphocytes T tumoricides qui
a) ont une action tumoricide vis-à-vis des lignées cellulaires ATCC CRL 1582 (MOLT-4), ATCC TIB 152 (Jurkat), ATCC TIB 202 (THP-1), ATCC CCL 240 (HL-60), ATCC CCL2 (Hela), ATCC CCL 243 (K-562), ATCC HTB 64 (Malme-3M) et ATCC HTB 18 (Y 79), et que
b) dans le surnageant de culture de ces lymphocytes T tumoricides, pendant la multiplication de ces cellules en présence de la lignée cellulaire DSM ACC 2122 (HB 654), pour une limite de détection de 0,5 UI/ml, des interleukines 2 ne sont pas détectables.

12. Procédé selon la revendication 11, caractérisé par le fait que comme préparation cellulaire, un utilise une préparation de cellules souches.

13. Procédé de préparation d'une fraction active de lignées cellulaires de lymphocytes, qui
a) dans une co-culture avec des lymphocytes, dans laquelle on évite une stimulation allogène, active les lymphocytes pour les transformer en lymphocytes T tumoricides, sans qu'il soit nécessaire d'ajouter des mitogènes ou des facteurs de croissance, et que
b) en sa présence, les lymphocytes ainsi activés se multiplient sans addition d'interleukine 2,
caractérisé par le fait qu'une lignée cellulaire de mammifère, qui présente ces propriétés,
(i) est fractionnée,
(ii) les fractions sont séparées et testées, pour vérifier si ces fractions activent les lymphocytes pour les transformer en lymphocytes T tumoricides de manière analogue à la lignée cellulaire initiale,
(iii) une telle fraction est sélectionnée et, après vérification de son activité, encore fractionnée et isolée jusqu'au degré de pureté voulu.

14. Procédé selon la revendication 13, caractérisé par le fait que, comme lignée cellulaire de mammifère, on utilise DSM ACC 2122 (HB 654) et DSM ACC 2166 (HB 617).

15. Procédé selon la revendication 13 ou 14, caractérisé par le fait que comme fraction active, on isole un éluat, une vésicule de membrane ou une vésicule infracellulaire.
